(19) Europäisches Patentamt
European Patent Office
Office européen des brevets

(11) Veröffentlichungsnummer: **0 401 507 A1**

# (12) EUROPÄISCHE PATENTANMELDUNG

(21) Anmeldenummer: **90108261.0**

(22) Anmeldetag: **30.04.90**

(51) Int. Cl.[5]: **C07K 7/64, A61K 37/43**

Die Bezeichnung der Erfindung wurde geändert (Richtlinien für die Prüfung im EPA, A-III, 7.3).

(30) Priorität: **11.05.89 DE 3915361**

(43) Veröffentlichungstag der Anmeldung: **12.12.90 Patentblatt 90/50**

(84) Benannte Vertragsstaaten: **AT BE CH DE DK ES FR GB IT LI NL SE**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Weber, Wolf-Dietrich, Dr.**
**Lindenweg 32**
**D-6107 Reinheim(DE)**
Erfinder: **Hölzemann, Günter, Dr.**
**Weedring 4**
**D-6104 Seeheim 1(DE)**
Erfinder: **Jonczyk, Alfred, Dr.**
**Scheppallee 57**
**D-6100 Darmstadt(DE)**
Erfinder: **Lues, Ingeborg, Dr.**
**Paul Wagner Strasse 13**
**D-6100 Darmstadt(DE)**
Erfinder: **Bartoszyk, Gerd**
**Heinrich-Fulda-Weg 22**
**D-6100 Darmstadt(DE)**
Erfinder: **Greiner, Hartmut, Dr.**
**Dieburgerstrasse 218**
**D-6100 Darmstadt(DE)**

## (54) Cyclische Tachykinin-Agonisten.

(57) Die Erfindung betrifft neue Cyclopeptide der Formel I

$$\left[ (NR^7\text{-}CHR^1\text{-}CO\text{-}NR^8\text{-}CHR^2\text{-}CO\text{-}NH\text{-}CHR^3\text{-}CO\text{-}NH\text{-}CHR^4\text{-}CO\text{-}NH\text{-}CHR^5\text{-}CO\text{-}NR^9\text{-}CHR^6\text{-}CO)_n \right] \quad I$$

worin $R^1$ bis $R^9$ und n die in Anspruch 1 angegebene Bedeutungen haben. Diese Substanzen wirken u.a. vasodilatierend und/oder analgetisch.

EP 0 401 507 A1

## Cyclopeptide

Die Erfindung betrifft neue Cyclopeptide der Formel I

$$\left[-(NR^7-CHR^1-CO-NR^8-CHR^2-CO-NH-CHR^3-CO-NH-CHR^4-CO-NH-CHR^5-CO-NR^9-CHR^6-CO)_n-\right] \quad I$$

worin

n 1 oder 2,

$R^1$ und $R^7$ jeweils H, A, Alkenyl oder Alkinyl mit jeweils bis zu 4 C-Atomen, Ar-alkyl, Het-alkyl, Cycloalkyl mit 3-7 C-Atomen, oder Cycloalkylalkyl mit 4-11 C-Atomen,

$R^1$ und $R^7$ zusammen auch unsubstituiertes oder durch A, Ar, Ar-alkyl, OH, OA oder Het-alkyl substituiertes Alkylen oder Alkenylen mit jeweils 2-6 C-Atomen,

$R^2$ A,

$R^3$ A, A-$S(O)_m$-alkyl, A-O-alkyl, Ar-alkyl oder Carbamoyl-alkyl,

$R^4$ H, A, $H_2N$-alkyl, HAN-alkyl, $A_2N$-alkyl oder, falls $R^1$ von H verschieden ist und/oder mindestens einer der Reste $R^2$, $R^3$, $R^5$ und/oder $R^6$ die R-Konfiguration besitzt, auch Carbamoyl-alkyl,

$R^5$ Ar-alkyl oder Het-alkyl,

$R^6$ A oder Ar-alkyl,

$R^8$ und $R^9$ jeweils H oder A,

A Alkyl mit 1-8 C-Atomen,

Ar unsubstituiertes oder ein- oder mehrfach durch A, OA, Hal, $CF_3$, OH und/oder $NH_2$ substituiertes Phenyl oder unsubstituiertes Naphthyl,

Het einen gesättigten oder ungesättigten 5- oder 6-gliedrigen heterocyclischen Rest mit 1-4 N-, O- und/oder S-Atomen, der mit einem Benzolring oder einem Pyridinring kondensiert und/oder ein- oder mehrfach durch A, OA, Hal, $CF_3$, OH, $NO_2$, Carbonylsauerstoff, $NH_2$, NHA, $NA_2$, NHAc, SA, SO-A, $SO_2$-A, COOA, CN, $CONH_2$, CONHA, $CONA_2$, CONHAr, CONH-alkyl-Ar, $SO_2NH_2$, $NHSO_2A$, Ar, Ar-alkyl, Ar-alkenyl, Hydroxy-alkyl und/oder Amino-alkyl mit jeweils 1-8 C-Atomen substituiert sein kann und/oder dessen N- und/oder S-Heteroatome auch oxydiert sein können,

-alkyl- eine Alkylenkette mit 1-4 C-Atomen,

-alkenyl- eine Alkenylenkette mit 2-4 C-Atomen,

Hal F, Cl, Br oder J,

m 0, 1 oder 2 und

Ac H-CO-, A-CO-, Ar-CO-, A-NH-CO- oder Ar-NH-CO-bedeuten,

sowie deren Salze.

Ähnliche Verbindungen sind aus Pharmazie 40 (8), 532-5, (1985) bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem wirken sie tachykinin-agonistisch (z. B. vasodilatierend) und/oder tachykinin-antagonistisch (z. B. analgetisch). Diese Wirkungen können z. B. nach den Methoden nachgewiesen werden, die in der US-A-4 472 305 angegeben sind, die vasodilatierende Wirkung z. B. auch nach der Methode von F. Lembeck et al., Biochem. Res. Comm. 103, 1318-1321 (1981), die analgetische Wirkung z. B. im Schleiftest an Ratten oder Mäusen (Methodik vgl. C. Vander Wende u. S. Margolin, Fed. Proc. 15, 494 ff. (1956); E. Siegmund et al., Proc. Soc. exp. Biol. (NY) 95, 729-731 (1957); L.L. Hendershot u. J. Forsaith, J. Pharmacol. exp. Ther. 125, 237-240 (1959)). Bei den Agonisten treten weiterhin Anregugen der Motorik und Blutdrucksenkungen, bei den Antagonisten antiinflammatorische und/oder spasmolytische Effekte auf. Weiterhin zeigen sich bei den Verbindungen der Formel I stimulierende Wirkungen auf die Tränensekretion, insbesondere bei lokaler Anwendung. Die Verbindungen der Formel I zeichnen sich außerdem aus durch große Selektivität und eine gute Stabilität gegen Proteasen.

Die Verbindungen können als Arzneimittelwirkstoffe in der Human- und Veterinärmedizin eingesetzt werden, insbesondere zur Prophylaxe und zur Behandlung von cardio vaskulären Erkrankungen, spastischen Erkrankungen, Schmerzzuständen, Entzündungen, Erkrankungen des Zentralnervensystems und/oder des Kreislaufs, und/oder zur Stimulierung der Tränensekretion.

Die vor- und nachstehend aufgeführten Abkürzungen von Aminosäureresten stehen für die Reste -NR-CR′R″-CO-(worin R, R′ und R″ die für jede Aminosäure bekannten spezifischen Bedeutungen haben) folgender Aminosäuren:
Ala Alanin
Gln Glutamin
Gly Glycin
His Histidin
Leu Leucin
Met Methionin
Met(O) Methionin-S-oxid
Met($O_2$) Methionin-S,S-dioxid
N-Me-Phe N-Methyl-phenylalanin
Phe Phenylalanin
Pro Prolin
Trp Tryptophan
Val Valin.

Ferner bedeutet nachstehend:
BOC tert.-Butoxycarbonyl
BOM Benzyloxymethyl
imi-BOM Benzyloxymethyl in 1-Stellung des Imidazolrings
CBZ Benzyloxycarbonyl
DCCI Dicyclohexylcarbodiimid
DMF Dimethylformamid
DNP 2,4-Dinitrophenyl
imi-DNP 2,4-Dinitrophenyl in 1-Stellung des Imidazolrings
EDCI N-Ethyl-N′-(3-dimethylaminopropyl)-carbodiimidhydrochlorid
FMOC 9-Fluorenylmethoxycarbonyl
HOBt 1-Hydroxybenzotriazol
Me Methyl
OMe Methylester
OEt Ethylester
POA Phenoxyacetyl
TFA Trifluoressigsäure

Sofern die vorstehend genannten Aminosäuren in mehreren enantiomeren Formen auftreten können, so sind vor- und nachstehend, z. B. als Bestandteil der Verbindungen der Formel I, alle diese Formen und auch ihre Gemische (z. B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur herstellung eines Cyclopeptids der Formel I, dadurch gekennzeichnet, daß man es aus einem seiner funktionellen Derivate durch Behandeln mit einem solvolysierenden oder hydrogenolysierenden Mittel in Freiheit setzt,
oder daß man ein Peptid der Formel II
H-$(Z)_n$-OH II
worin
n 1 oder 2 und
Z -$NR^7$-$CHR^1$-CO-$NR^8$-$CHR^2$-CO-NH-$CHR^3$-CO-NH-$CHR^4$-CO-NH-$CHR^5$-CO-$NR^9$-$CHR^6$-CO-,
-$NR^8$-$CHR^2$-CO-NH-$CHR^3$-CO-NH-$CHR^4$-CO-NH-$CHR^5$-CO-$NR^9$-$CHR^6$-CO-$NR^7$-$CHR^1$-CO-,
-NH-$CHR^3$-CO-NH-$CHR^4$-CO-NH-$CHR^5$-CO-$NR^9$-$CHR^6$-CO-$NR^7$-$CHR^1$-CO-$NR^8$-$CHR^2$-CO-,
-NH-$CHR^4$-CO-NH-$CHR^5$-CO-$NR^9$-$CHR^6$-CO-$NR^7$-$CHR^1$-CO-$NR^8$-$CHR^2$-CO-NH-$CHR^3$-CO-,
-NH-$CHR^5$-CO-$NR^9$-$CHR^6$-CO-$NR^7$-$CHR^1$-CO-$NR^8$-$CHR^2$-CO-NH-$CHR^3$-CO-NH-$CHR^4$-CO-
oder
-$NR^9$-$CHR^6$-CO-$NR^7$-$CHR^1$-CO-$NR^8$-$CHR^2$-CO-NH-$CHR^3$-CO-NH-$CHR^4$-CO-NH-$CHR^5$-CO-
bedeuten,
oder ein reaktionsfähiges Derivat eines solchen Peptids mit einem cyclisierenden Mittel behandelt, und daß man gegebenenfalls in einer Verbindung der Formel I eine Thioethergruppe zu einer Sulfoxidgruppe oder zu einer Sulfongruppe oxydiert und/oder eine Sulfoxidgruppe zu einer Thioethergruppe reduziert und/oder eine Verbindung der Formel I durch Behandeln mit einer Säure in eines ihrer Salze überführt.

Vor- und nachstehend haben die Reste bzw. Parameter n, $R^1$ bis $R^9$, A, Ar, Het, Hal, m, Ac und Z die bei den Formeln I oder II angegebenen Bedeutungen, falls nicht ausdrücklich etwas anderes angegeben ist.

In den vorstehenden Formeln hat A 1 - 8, vorzugsweise 1,2,3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2-oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z. B. 1-, 2- oder 3-Methylcyclopentyl, 1-, 2-, 3- oder 4-Methylcyclohexyl.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z. B. 1-, 2- oder 3-Methylcyclopentylmethyl, 1-, 2-, 3- oder 4-Methylcyclohexylmethyl.

Hal bedeutet vorzugsweise F, Cl oder Br, aber auch J.

Ac bedeutet vorzugsweise H-CO-, A-CO- wie Acetyl, Propionyl oder Butyryl, Ar-CO- wie Benzoyl, o-, m- oder p-Methoxybenzoyl oder 3,4-Dimethoxybenzoyl, A-NH-CO-wie N-Methyl- oder N-Ethylcarbamoyl, Ar-NH-CO- wie N-Phenylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m-oder p-Hydroxyphenyl, 2,3-, 2,4-, 2,5-, 2,6-, 3,4- oder 3,5-Dimethoxyphenyl, 3,4,5-Trimethoxyphenyl, o-, m- oder p-Aminophenyl, 1- oder 2-Naphthyl.

Dementsprechend bedeutet Ar-alkyl vorzugsweise Benzyl, 1- oder 2-Phenylethyl, o-, m- oder p-Methylbenzyl, 1-oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1-oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m- oder -p-Jodphenylethyl, o-, m- oder p-Trifluormethylbenzyl, o-, m- oder p-Hydroxybenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, 1- oder 2-Naphthylmethyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4-oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5- 6- oder 7-Isoindolyl, 1-, 2-, 4-, oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6-, 7- oder 8-Chinazolyl.

Die heterocyclischen Reste können auch teilweise oder vollständig hydriert sein. Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, Tetrahydro-2- oder -3-thienyl, 2,3-Dihydro-1-, -2-, -3-, -4- oder -5-pyrryl, 2,5-Dihydro-1-, -2-, -3-, -4- oder -5-pyrryl, 1-, 2- oder 3-Pyrrolidinyl, Tetrahydro-1-, -2- oder -4-imidazolyl, 2,3-Dihydro-1-, -2-, -3-, -4- oder 5-pyrazolyl, 2,5-Dihydro-1-, -2-, -3-, -4-oder 5-pyrazolyl, Tetrahydro-1-, -3-, oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidyl, 1-, 2- oder 3-Piperazinyl, 1,2,3,4-Tetrahydro-1-, -2-, -3-, -4-, -5-, -6-, -7-, oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann also bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 2-Amino-5,6-dimethyl-3-pyrazinyl, 4-Carbamoylpiperidino, ferner z. B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrrolyl, 1-Methyl-4- oder -5-nitro-2-pyrrolyl, 3,5-Dimethyl-4-ethyl-2-pyrrolyl, 4-Methyl-5-pyrazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl,

2,6-Dichlorpyridyl, 2-Hydroxy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N′-Methylureido-1H-4-pyridon-5-yl, 5- oder 6-Methyl-4-pyrimidyl, 2,6-Dihydroxy-4-pyrimidyl, 5-Chlor-2-methyl-4-pyrimidyl, 2-Methyl-4-amino-5-pyrimidyl, 3-Methyl-2-benzofuryl, 2-Ethyl-3-benzofuryl, 7-Methyl-2-benzothienyl, 1-, 2-, 4-, 5-, 6- oder 7-Methyl-3-indolyl, 1-Methyl-5- oder -6-benzimidazolyl, 1-Ethyl-5- oder -6-benzimidazolyl, 3-, 4-, 5-, 6-, 7- oder 8-Hydroxy-2-chinolyl.

$R^1$ und $R^7$ bedeuten vorzugsweise jeweils H oder A oder zusammen eine Alkylenkette mit 2-6, insbesondere 3 C-Atomen.

$R^2$ bedeutet bevorzugt Propyl, Isobutyl oder sek.-Butyl.

$R^3$ ist bevorzugt A-S(O)$_m$-alkyl, insbesondere $CH_3$-S(O)$_m$-$CH_2CH_2$-, wobei m bevorzugt 0 ist.

$R^4$ ist bevorzugt H oder (falls $R^1$ von H verschieden ist und/oder mindestens einer der Reste $R^2$ bis $R^6$ die R-Konfiguration besitzt) $H_2N$-CO-$CH_2CH_2$-.

$R^5$ ist vorzugsweise Ar-alkyl oder Het-alkyl, insbesondere Benzyl oder 3-Indolyl-methyl, ferner p-Hydroxybenzyl oder p-Methoxybenzyl.

$R^6$ ist bevorzugt Ar-alkyl, insbesondere Benzyl, ferner p-Hydroxybenzyl oder p-Methoxybenzyl.

Vorzugsweise bedeutet:

-N$R^7$-CH$R^1$-CO-: Gly, Pro oder D-Pro;

-N$R^8$-CH$R^2$-CO-: Leu, ferner D-Leu, Ile oder D-Ile;

-NH-CH$R^3$-CO-: Met, D-Met, Met(O), D-Met(O), Met($O_2$) oder D-Met($O_2$), ferner Leu oder D-Leu;

-NH-CH$R^4$-CO-: Gly oder Gln, ferner Ala oder D-Ala;

-NH-CH$R^5$-CO-: Phe oder Trp;

-N$R^9$-CH$R^6$-CO-: Phe, D-Phe, N-Me-Phe oder D-N-Me-Phe, ferner Val oder D-Val.

Der Parameter n ist vorzugsweise 1.

Dementsprechend sind Gegenstand der Erfindung insbesondere diejenigen Verbindungen der Formel I, in denen mindestens einer der genannten Reste und/oder Parameter eine der vorstehend angegebenen bevorzugten Bedeutungen hat. Einige bevorzugte Gruppen von Verbindungen können durch die Teilformel Ia ausgedrückt werden, die sonst der Formel I entspricht, worin jedoch

$R^1$ H oder zusammen mit $R^7$ -$(CH_2)_3$-,

$R^2$ Isobutyl,

$R^3$ $CH_3$-S(O)$_m$-$CH_2CH_2$-,

$R^4$ H oder, falls $R^1$ von H verschieden ist und/oder mindestens einer der Reste $R^2$, $R^3$, $R^4$, $R^5$ und/oder $R^6$ die R-Konfiguration besitzt, auch $H_2N$-CO-$CH_2CH_2$-,

$R^5$ Benzyl oder 3-Indolyl-methyl,

$R^6$ Benzyl,

$R^7$ H oder zusammen mit $R^1$ -$(CH_2)_3$-,

$R^8$ H und

$R^9$ H oder Me bedeuten.

Besonders bevorzugt sind Verbindungen der Formeln I und Ia, worin zusätzlich n = 1 bedeutet.

Die Verbindungen der Formel I und auch die Ausgangsstoffe zu ihrer Herstellung werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino- und/oder Hydroxygruppen entsprechende geschützte Amino- und/oder hydroxygruppen enthalten, vorzugsweise solche, die an Stelle eines H-Atoms, das mit einem N-Atom verbunden ist, eine Aminoschutzgruppe tragen, z. B. solche, die der Formel I entsprechen, aber an Stelle einer His-Gruppe eine N(im)-R′-His-Gruppe (worin R′ eine Aminoschutzgruppe bedeutet, z. B. BOM oder DNP) enthalten.

Ferner sind Ausgangsstoffe bevorzugt, die an Stelle des H-Atoms einer Hydroxygruppe eine Hydroxyschutzgruppe tragen, z. B. solche, die der Formel I entsprechen, aber anstelle einer Hydroxyphenylgruppe eine R″O-phenylgruppe enthalten (worin R″ eine Hydroxyschutzgruppe bedeutet).

Es können auch mehrere - gleiche oder verschiedene -geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls duchgeführt worden ist. Typisch für solche Gruppen sind insbesondere unsubstituierte oder substituierte Acyl-, Aryl- (z. B. DNP), Aralkoxymethyl- (z. B. BOM) oder Aralkylgruppen (z. B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z. B. in den genannten Standardwerken und Patentanmeldungen beschrieben sind, z. B. auch nach der Festphasenmethode nach Merrifield.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser.

Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z. B. bevorzugt mit 40%iger TFA in Dichlormethan oder mit etwa 3 bis 5 Ⓝ HCl in Dioxan bei 15-30° abgespalten werden, die FMOC-Gruppe mit einer etwa 5- bis 20%igen Lösung von Dimethylamin, Diethylamin oder Piperidin in DMF bei 15-30°. Eine Abspaltung der DNP-Gruppe gelingt z. B. auch mit einer etwa 3-bis 10%igen Lösung von 2-Mercaptoethanol in DMF/Wasser bei 15-30°.

Hydrogenolytisch entfernbare Schutzgruppen (z. B. BOM, CBZ oder Benzyl) können z. B. durch Behandeln mit Wasserstoff in gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5- bis 10 %igem Pd-C in methanol oder mit Ammoniumformiat (an Stelle von $H_2$) an Pd-C in Methanol/DMF bei 20-30°.

Verbindungen der Formel I können auch durch Cyclisierung von Verbindungen der Formel II unter den Bedingungen einer Peptidsynthese erhalten werden. Dabei arbeitet man zweckmäßig nach üblichen Methoden der Peptid-Synthese, wie sie z. B. in Houben-Weyl, 1.c., Band 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder EDCI, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, oder in Gemischen dieser Lösungsmittel, bei Temperaturen zwischen etwa -10 und 40, vorzugsweise zwischen 0 und 30°. Um die intramolekulare Cyclisierung vor der intermolekularen Peptid-Bindung zu fördern, ist es zweckmäßig, in verdünnten Lösungen zu arbeitet (Verdünnungsprinzip).

Anstelle von II können auch geeignete reaktionsfähige Derivate dieser Stoffe in die Reaktion eingesetzt werden, z. B. solche, in denen reaktive Gruppen intermediär durch Schutzgruppen blockiert sind. Die Aminosäurederivate II können z. B. in Form ihrer aktivierten Ester verwendet werden, die zweckmäßig in situ gebildet werden, z. B. durch Zusatz von HOBt oder N-Hydroxysuccinimid.

Die Ausgangsstoffe der Formel II sind in der Regel neu. Sie können nach bekannten Methoden, z. B. den oben angegebenen Methoden der Peptidsynthese und der Abspaltung von Schutzgruppen, hergestellt werden.

In der Regel synthetisiert man zunächst geschützte Hexapeptidester der Formel R′-Z-OR″, z. B. BOC-Z-OMe oder BOC-Z-OEt, die zunächst zu Säuren der Formel R′-Z-OH, z. B. BOC-Z-OH verseift werden; aus diesen wird die Schutzgruppe R′ abgespalten, wodurch man die freien Peptide der Formel H-Z-OH (II; n = 1) erhält. Die Dodekapeptide der Formel $H-(Z)_2-OH$ werden zweckmäßig durch Umsetzung von Säuren der Formel R′-Z-OH mit Aminoestern der Formel H-Z-OR″ zu geschützten Dodekapeptiden der Formel $R'-(Z)_2-OR''$ sowie nachfolgende Abspaltung der Schutzgruppen R′ und R″ hergestellt.

Es ist auch möglich, eine Thioethergruppe zu einer Sulfoxidgruppe oder Sulfongruppe , insbesondere eine Gruppe $-NH-CHR^3-CO-$ = Met zu einer Gruppe oder $-NH-CHR^3-CO-$ = Met(O) oder $Met(O_2)$ zu oxydieren, z. B. durch Einleiten von Luft in eine Lösung der Verbindung der Formel I ($-NH-CHR^3-CO-$ = Met) in Acetonitril/Wasser bei Temperaturen zwischen 0 und 30 °. Man kann den Thioether auch mit $H_2O_2$ oxydieren. So erhält man in Methanol mit der berechneten Menge Oxydationsmittel bei 20° überwiegend das Sulfoxid, mit einem Überschuß des Oxydationsmittels bei 50° dagegen überwiegend das Sulfon.

Umgekehrt kann man eine Sulfoxidgruppe (z. B. in I, $-NH-CHR^3-CO-$ = Met(O)) zu einer Thioethergruppe (z. B. in I, $-NH-CHR^3-CO-$ = Met) reduzieren z. B.mit $NH_4J$ in wässeriger TFA bei Temperaturen zwischen -10 und 25°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z. B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zübereitungen können als Arzneimittel in der Human- oder Veterinärmedizin eingesetzt werden. Als Trägersubstanzen kommen organische oder anorganische Stoffe in Frage, die sich für die enterale (z. B. orale oder rektale), parenterale oder lokale (z. B. topische) Applikation oder für eine Applikation in Form eines Inhalations-Sprays eignen und mit den neuen Verbindungen nicht reagieren, beispielsweise Wasser, niedere Alkohole, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose, Vaseline. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Zur topischen Anwendung eignen sich z. B. Lösungen, die in Form von Augentropfen verwendet werden können, ferner z. B. Suspensionen, Emulsionen, Cremes, Salben oder Komprimate. Für die Applikation als Inhalations-Spray können Sprays verwendet werden, die den Wirkstoff entweder gelöst oder suspendiert in einem Treibgasgemisch (z. B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit hilfe üblicher Inhalatoren verabfolgt werden. Die neuen Verbindungen können auch lyophilisiert und die erhaltenen Lyophilisate z. B. zur Herstellung von Injektionspräparaten verwendet werden. Die angegebenen Zübereitungen können sterilisiert sein und/oder Hilfsstoffe wie Konservierungs-, Stabilisierungs-und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine.

Die erfindungsgemäßen Substanzen werden in der Regel in Analogie zu anderen bekannten, im Handel befindlichen Peptiden, insbesondere aber in Analogie zu den in der US-A-4 472 305 beschriebenen Verbindungen verabreicht, vorzugsweise in Dosierungen zwischen etwa 0,05 und 500, insbesondere zwischen 0,5 und 100 mg pro Dosierungseinheit. Die tägliche Dosierung liegt vorzugsweise zwischen etwa 0,01 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation. RZ = Retentionszeit (Minuten) bei HPLC an RP 18 250-4-Säule, wenn nicht anders angegeben; Laufmittel: a = Wasser, b = 0,3 % TFA in Wasser, c = Acetonitril. Rf = Rf-Wert dünnschichtchromatographisch an Kieselgel; Laufmittel: Chloroform/Methanol/Essigsäure 85:10:5. FAB-MS = "fast atom bombardment"-Massenspektrum.

Beispiel 1

Ein Gemisch von 900 mg Cyclo-[-glycyl-L-phenylalanyl-L-phenylalanyl-L-(N-imi-2,4-dinitrophenyl-histidyl)-L-leucyl-L-methionin-] [Cyclo-(-Gly-Phe-Phe-(imi-DNP-His)-Leu-Met-); erhältlich durch Cyclisierung von H-Gly-Phe-Phe-(imi-DNP-His)-Leu-Met-OH analog Beispiel 3, s. unten], 2 g 2-Mercaptoethanol, 20 ml DMF und 20 ml Wasser wird unter Rühren bei 20° mit wässeriger $Na_2CO_3$-Lösung auf pH 8 eingestellt und 2 Std. bei 20° gerührt. Nach üblicher Aufarbeitung erhält man Cyclo-(-glycyl-L-phenylalanyl-L-phenylalanyl-L-histidyl-L-leucyl-L-methionin-)[Cyclo-(-Gly-Phe-Phe-His-Leu-Met-)].

Beispiel 2

Man löst 1 g Cyclo-[-Gly-Phe-Phe-(imi-BOM-His)-Leu-Met-] [erhältlich aus H-Gly-Phe-Phe-(imi-BOM-His)-Leu-Met-OH analog Beispiel 3, s. unten] in 25 ml Methanol, hydriert 3 Std. an 0,5 %ig. Pd-C bei 20° und 1 bar, filtriert, dampft ein und erhält nach üblicher Aufarbeitung Cyclo-(-Gly-Phe-Phe-His-Leu-Met-).

Beispiel 3

Man löst 1,8 g (2 mmol) BOC-Leu-D-Met-Gln-Trp-Phe-Gly-OMe in 50 ml Methanol, versetzt mit 1,5 ml 2 n wässeriger Natronlauge und rührt 3 Std. bei 20°. Man dampft ein, nimmt in Wasser auf, säuert mit HCl bis pH 3 an und extrahiert mit Ethylacetat. Der Extrakt wird über $Na_2SO_4$ getrocknet und eingedampft. Das so erhaltene BOC-Leu-D-Met-Gln-Trp-Phe-Gly-OH wird mit 20 ml 2 n HCl in Dioxan 2 Std. bei 20° gerührt. Man dampft ein, löst das erhaltene H-Leu-D-Met-Gln-Trp-Phe-Gly-OH in einem Gemisch von 1800 ml Dichlormethan und 200 ml DMF, kühlt auf 0°, gibt unter Rühren nacheinander 0,5 g DCCI, 0,3 g HOBt und 0,23 ml N-Methylmorpholin hinzu, rührt 24 Std. bei 0° und 48 Std. bei 20°. Die Lösung wird eingeengt und zur Entfernung von Salzen mit einem Mischbett-Ionenaustauscher gerührt. Dieser wird abfiltriert, die Lösung eingedampft und der Rückstand chromatographisch gereinigt. Man erhält Cyclo-(-Gln-Trp-Phe-Gly-Leu-D-Met-); RZ 11,87 (b/c 6:4).

Analog erhält man aus den entsprechenden linearen Hexapeptiden:

Cyclo-(-Ala-Phe-Phe-Pro-Leu-Met-), F. 134-140 °
Cyclo-[-Ala-Phe-Phe-Pro-Leu-Met(O)-]
Cyclo-[-Ala-Phe-Phe-Pro-Leu-Met($O_2$)-]
Cyclo-(-Ala-Phe-Phe-Pro-Leu-D-Met-), F. 133-138 °
Cyclo-(-Gly-Phe-N-Me-Phe-Gly-Leu-Met-), RZ 15,09 (a/c 6:4)
Cyclo-[-Gly-Phe-N-Me-Phe-Gly-Leu-Met(O)-]
Cyclo-[-Gly-Phe-N-Me-Phe-Gly-Leu-Met($O_2$)-]
Cyclo-(-Gly-Phe-D-N-Me-Phe-Gly-Leu-Met-), RZ 12,30 (a/c 6:4)
Cyclo-[-Gly-Phe-D-N-Me-Phe-Gly-Leu-Met(O)-]
Cyclo-[-Gly-Phe-D-N-Me-Phe-Gly-Leu-Met($O_2$)-]
Cyclo-(-Gly-Phe-Phe-Pro-Leu-Leu-)
Cyclo-(-Gly-Phe-Phe-Pro-Leu-Met-), FAB-MS: $(M+H)^+$ 694
Cyclo-[-Gly-Phe-Phe-Pro-Leu-Met(O)-], FAB-MS: $(M+H)^+$ 710
Cyclo-[-Gly-Phe-Phe-Pro-Leu-Met($O_2$)-], F. 157-162°
Cyclo-(-Gly-Phe-D-Phe-Pro-Leu-Met-)
Cyclo-[-Gly-Phe-D-Phe-Pro-Leu-Met(O)-]
Cyclo-[-Gly-Phe-D-Phe-Pro-Leu-Met($O_2$)-], F. 128-135 °
Cyclo-(-Gly-Phe-Phe-D-Pro-Leu-Met-), Rf 0,63
Cyclo-[-Gly-Phe-Phe-D-Pro-Leu-Met(O)-], Rf 0,42
Cyclo-[-Gly-Phe-Phe-D-Pro-Leu-Met($O_2$)-]
Cyclo-(-Gly-Phe-Phe-Pro-Leu-D-Met-), F. 134-140 °
Cyclo-[-Gly-Phe-Phe-Pro-Leu-D-Met(O)-]
Cyclo-[-Gly-Phe-Phe-Pro-Leu-D-Met($O_2$)-]
Cyclo-(-Gly-Phe-Val-Gly-Leu-Met-), Rf 0,51
Cyclo-[-Gly-Phe-Val-Gly-Leu-Met(O)-]
Cyclo-[-Gly-Phe-Val-Gly-Leu-Met($O_2$)-]
Cyclo-(Gly-Trp-Phe-Pro-Leu-Met-)
Cyclo-[Gly-Trp-Phe-Pro-Leu-Met(O)-]
Cyclo-[Gly-Trp-Phe-Pro-Leu-Met($O_2$)-]
Cyclo-(Gly-Trp-Phe-D-Pro-Leu-Met-)
Cyclo-[Gly-Trp-Phe-D-Pro-Leu-Met(O)-]
Cyclo-[Gly-Trp-Phe-D-Pro-Leu-Met($O_2$)-].

Beispiel 4

Analog Beispiel 3 erhält man durch Cyclisierung von H-(Gly-Phe-Phe-Pro-Leu-Met)$_2$-OH [erhältlich durch Reaktion von BOC-Gly-Phe-Phe-Pro-Leu-Met-OH mit H-Gly-Phe-Phe-Pro-Leu-Met-OMe zu BOC-(Gly-Phe-Phe-Pro-Leu-Met)$_2$-OMe und nachfolgende Abspaltung der Schutzgruppe] das Cyclo-(-Gly-Phe-Phe-Pro-Leu-Met-)$_2$, F. 216-218°.

Analog erhält man aus den entsprechenden linearen Dodekapeptiden:
Cyclo-(-Ala-Phe-Phe-Pro-Leu-Met-)$_2$, F. 137-144 °
Cyclo-(-Ala-Phe-Phe-Pro-Leu-D-Met)$_2$, Rf 0,74
Cyclo-[-Gly-Phe-Phe-Pro-Leu-Met(O)-]$_2$
Cyclo-[-Gly-Phe-Phe-Pro-Leu-Met($O_2$)-]$_2$
Cyclo-(-Gly-Trp-Phe-Pro-Leu-Met-)$_2$
Cyclo-[-Gly-Trp-Phe-Pro-Leu-Met(O)-]$_2$
Cyclo-[-Gly-Trp-Phe-Pro-Leu-Met-($O_2$)-]$_2$
Cyclo-(-Gly-Trp-Phe-D-Pro-Leu-Met-)$_2$
Cyclo-[-Gly-Trp-Phe-D-Pro-Leu-Met-(O)-]$_2$
Cyclo-[-Gly-Trp-Phe-D-Pro-Leu-Met-($O_2$)-]$_2$

Beispiel 5

Man löst 1,7 g (2 mmol) BOC-Gly-Phe-Phe-Pro-Leu-Met-OMe (F. 92-99°) in 50 ml Methanol, versetzt mit 2 ml Hydrazinhydrat, rührt 16 Std. und dampft ein. Das so erhaltene BOC-Gly-Phe-Phe-Pro-Leu-Met-$NHNH_2$ wird mit 20 ml 2 n HCl in Dioxan 2 Std. bei 20° gerührt. Man dampft ein, löst das erhaltene H-Gly-Phe-Phe-Pro-Leu-Met-$NHNH_2$-dihydrochlorid in 50 ml DMF, kühlt unter Rühren auf -15° und gibt nacheinander 0,83 ml 35%ige Salzsäure und 1,5 ml einer 14%igen wässerigen $NaNO_2$-Lösung hinzu. Es wird 1 Std. bei -15° gerührt und in 2 l gekühltes DMF gegossen. Man versetzt mit 1,1 ml N-Methylmorpholin, rührt 24 Std bei -15° und läßt 2 Tage bei 20° stehen. Man kon zentriert die Lösung, versetzt zur Entfernung von Salzen mit einem Mischbett-Ionenaustauscher, filtriert, dampft das Filtrat ein, reinigt chromatographisch und erhält Cyclo-(-Gly-Phe-Phe-Pro-Leu-Met-); FAB-MS $(M+H)^+$ 694.

Beispiel 6

Eine Lösung von 100 mg Cyclo-(-Gly-Phe-Phe-Pro-Leu-Met-) und 1 Äquivalent 30%ig. wässerigem $H_2O_2$ in 50 ml Methanol wird 2 Std. bei 20° gerührt. Nach Eindampfen und üblicher Aufarbeitung erhält man Cyclo-[-Gly-Phe-Phe-Pro-Leu-Met(O)-]; FAB-MS: $(M+H)^+$ 710.

Beispiel 7

Eine Lösung von 100 mg Cyclo-(-Gly-Phe-Phe-Pro-Leu-Met-) und 10 Äquivalenten 30%ig. wässerigem $H_2O_2$ in 50 ml Methanol wird 2 Std. auf 50° erwärmt. Nach Eindampfen und üblicher Aufarbeitung erhält man Cyclo-[-Gly-Phe-Phe-Pro-Leu-Met($O_2$)-]; F. 157-162°.

Beispiel 8

Man leitet Luft durch eine Losung von 1 g Cyclo-(-Gly-Phe-Phe-Pro-Leu-Met-) in 50 ml Acetonitril und 50 ml Wasser bis zur vollständigen Umsetzung. Nach ublicher Aufarbeitung erhält man das entsprechende Sulfoxid Cyclo-[-Gly-Phe-Phe-Pro-Leu-Met(O)-]; FAB-MS: $(M+H)^+$ 710.

Beispiel 9

Eine Lösung von 1 g Cyclo-[-Gly-Phe-Phe-Pro-Leu-Met(O)-] in 50 ml TFA wird bei 0° mit 20 ml 2 m $NH_4J$-Lösung versetzt. Nach 1 Std. Rühren bei 0° reduziert man das entstandene Jod durch Zugabe von Thioglykolsäure, arbeitet wie üblich auf und erhält Cyclo-(-Gly-Phe-Phe-Pro-Leu-Met-); FAB-MS: $(M+H)^+$ 694.

Die nachstehenden Beispiele betreffen pharmazeutische Zübereitungen.

Beispiel A: Injektionsgläser

Eine Lösung von 100 g Cyclo-(-Gly-Phe-Phe-Pro-Leu-Met-) und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

Beispiel B: Suppositorien

Man schmilzt ein Gemisch von 20 g Cyclo-(-Gln-Trp-Phe-Gly-Leu-D-Met-) mit 100 g Sojalecithin und 1400 g Kakaobutter, gießt in Formen und läßt erkalten. Jedes Suppositorium enthält 20 mg Wirkstoff.

Beispiel C: Lösung

Man bereitet eine Lösung aus 1 g Cyclo-[-Gly-Phe-Phe-Pro-Leu-Met(O)-], 9,38 g $NaH_2PO_4$ . 2 $H_2O$, 28,48 g $Na_2HPO_4$ . 12 $H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

Beispiel D: Salbe

Man mischt 500 mg Cyclo-(-Gly-Phe-Phe-Pro-Leu-Met-)$_2$ mit 99,5 g Vaseline unter aseptischen Bedingungen.

## Ansprüche

1. Cyclopeptide der Formel I

$$\text{cyclo}\left[-(NR^7\text{-}CHR^1\text{-}CO\text{-}NR^8\text{-}CHR^2\text{-}CO\text{-}NH\text{-}CHR^3\text{-}CO\text{-}NH\text{-}CHR^4\text{-}CO\text{-}NH\text{-}CHR^5\text{-}CO\text{-}NR^9\text{-}CHR^6\text{-}CO)_n-\right] \quad I$$

worin
n 1 oder 2,
$R^1$ und $R^7$ jeweils H, A, Alkenyl oder Alkinyl mit jeweils bis zu 4 C-Atomen, Ar-alkyl, Hetalkyl, Cycloalkyl mit 3-7 C-Atomen, oder Cycloalkylalkyl mit 4-11 C-Atomen,
$R^1$ und $R^7$ zusammen auch unsubstituiertes oder durch A, Ar, Ar-alkyl, OH, OA oder Het-alkyl substituiertes Alkylen oder Alkenylen mit jeweils 2-6 C-Atomen,
$R^2$ A,
$R^3$ A, A-S(O)$_m$-alkyl, A-O-alkyl, Ar-alkyl oder Carbamoyl-alkyl,
$R^4$ H, A, $H_2N$-alkyl, HAN-alkyl, $A_2N$-alkyl oder, falls $R^1$ von H verschieden ist und/oder mindestens einer der Reste $R^2$, $R^3$, $R^5$ und/oder $R^6$ die R-Konfiguration besitzt, auch Carbamoyl-alkyl,
$R^5$ Ar-alkyl oder Het-alkyl,
$R^6$ A oder Ar-alkyl,
$R^8$ und $R^9$ jeweils H oder A,
A Alkyl mit 1-8 C-Atomen,
Ar unsubstituiertes oder ein- oder mehrfach durch A, OA, Hal, $CF_3$, OH und/oder $NH_2$ substituiertes Phenyl oder unsubstituiertes Naphthyl,
Het einen gesättigten oder ungesättigten 5-oder 6-gliedrigen heterocyclischen Rest mit 1-4 N-, O- und/oder S-Atomen, der mit einem Benzolring oder einem Pyridinring kondensiert und/oder ein- oder mehrfach durch A, OA, Hal, $CF_3$, OH, $NO_2$, Carbonylsauerstoff, $NH_2$, NHA, $NA_2$, NHAc, SA, SO-A, $SO_2$-A, COOA, CN, $CONH_2$, CONHA, $CONA_2$, CONHAr, CONH-alkyl-Ar, $SO_2NH_2$, $NHSO_2A$, Ar, Ar-alkyl, Ar-alkenyl, Hydroxy-alkyl und/oder Amino-alkyl mit jeweils 1-8 C-Atomen substituiert sein kann und/oder dessen N- und/oder S-Heteroatome auch oxydiert sein können,
-alkyl- eine Alkylenkette mit 1-4 C-Atomen,
-alkenyl- eine Alkenylenkette mit 2-4 C-Atomen,
Hal F, Cl, Br oder J,
m 0, 1 oder 2 und
Ac H-CO-, A-CO-, Ar-CO-, A-NH-CO- oder Ar-NH-CO-
bedeuten,
sowie deren Salze.

2.
a) Cyclo-(-Gly-Phe-Phe-Pro-Leu-Met-);
b) Cyclo-[-Gly-Phe-Phe-Pro-Leu-Met(O)-];
c) Cyclo-(-Gly-Phe-Phe-Pro-Leu-Met-)$_2$.

3. Verfahren zur Herstellung eines Cyclopeptids der Formel I nach Anspruch 1, dadurch gekennzeichnet, daß man es aus einem seiner funktionellen Derivate durch Behandeln mit einem solvolysierenden oder hydrogenolysierenden Mittel in Freiheit setzt,
oder daß man ein Peptid der Formel II
H-(Z)$_n$-OH II
worin
n 1 oder 2 und
Z $-NR^7\text{-}CHR^1\text{-}CO\text{-}NR^8\text{-}CHR^2\text{-}CO\text{-}NH\text{-}CHR^3\text{-}CO\text{-}NH\text{-}CHR^4\text{-}CO\text{-}NH\text{-}CHR^5\text{-}CO\text{-}NR^9\text{-}CHR^6\text{-}CO\text{-}$,
$-NR^8\text{-}CHR^2\text{-}CO\text{-}NH\text{-}CHR^3\text{-}CO\text{-}NH\text{-}CHR^4\text{-}CO\text{-}NH\text{-}CHR^5\text{-}CO\text{-}NR^9\text{-}CHR^6\text{-}CO\text{-}NR^7\text{-}CHR^1\text{-}CO\text{-}$,
$-NH\text{-}CHR^3\text{-}CO\text{-}NH\text{-}CHR^4\text{-}CO\text{-}NH\text{-}CHR^5\text{-}CO\text{-}NR^9\text{-}CHR^6\text{-}CO\text{-}NR^7\text{-}CHR^1\text{-}CO\text{-}NR^8\text{-}CHR^2\text{-}CO\text{-}$,
$-NH\text{-}CHR^4\text{-}CO\text{-}NH\text{-}CHR^5\text{-}CO\text{-}NR^9\text{-}CHR^6\text{-}CO\text{-}NR^7\text{-}CHR^1\text{-}CO\text{-}NR^8\text{-}CHR^2\text{-}CO\text{-}NH\text{-}CHR^3\text{-}CO\text{-}$,

$-NH-CHR^5-CO-NR^9-CHR^6-CO-NR^7-CHR^1-CO-NR^8-CHR^2-CO-NH-CHR^3-CO-NH-CHR^4-CO-$
oder
$-NR^9-CHR^6-CO-NR^7-CHR^1-CO-NR^8-CHR^2-CO-NH-CHR^3-CO-NH-CHR^4-CO-NH-CHR^5-CO-$
bedeuten,
oder ein reaktionsfähiges Derivat eines solchen Peptids mit einem cyclisierenden Mittel behandelt, und daß man gegebenenfalls in einer Verbindung der Formel I eine Thioethergruppe zu einer Sulfoxidgruppe oder zu einer Sulfongruppe oxydiert und/oder eine Sulfoxidgruppe zu einer Thioethergruppe reduziert und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihres physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels.

7. Verwendung von Verbindungen der Formel I oder von deren physiologisch unbedenklichen Salzen bei der Bekämpfung von cardiovasculären Erkrankungen, spastischen Erkrankungen, Schmerzzuständen, Entzündungen, Erkrankungen des Zentralnervensystems und/oder des Kreislaufs und/oder bei der Stimulierung der Tränensekretion.

Europäisches Patentamt

# EUROPÄISCHER RECHERCHENBERICHT

Nummer der Anmeldung

EP 90 10 8261

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DIE PHARMAZIE, Band 40, Nr. 7, Juli 1985, Seiten 546-549, VEB Verlag Volk und Gesundheit, Berlin, DD; M. BORNSCHEIN et al.: "Entwicklung und gegenwärtiger Stand der Methoden zur Bestimmung der in-vitro-Arzneistoffverfügbarkeit von Suppositorien" --- | | C 07 K 7/64<br>A 61 K 37/43 |
| P,X | GB-A-2 216 529 (MERCK SHARP & DOHME LTD)<br>* Anspruch 1 *<br>----- | 1 | |
| | | | RECHERCHIERTE SACHGEBIETE (Int. Cl.5) |
| | | | C 07 K<br>A 61 K |

Der vorliegende Recherchenbericht wurde für alle Patentansprüche erstellt

| Recherchenort | Abschlußdatum der Recherche | Prüfer |
|---|---|---|
| DEN HAAG | 10-08-1990 | DEFFNER C-A.E. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)